(19) Europäisches Patentamt
European Patent Office
Office européen des brevets

(11) Veröffentlichungsnummer: **0 002 734**
**B1**

(12) # EUROPÄISCHE PATENTSCHRIFT

(21) Anmeldenummer: **78101675.3**

(22) Anmeldetag: **14.12.78**

(51) Int. Cl.³: **C 07 D 317/60,**
C 07 D 405/10

(54) **Verfahren zur Herstellung von Piperonylidencrotonsäureamiden**

(30) Priorität: **22.12.77 DE 2757483**

(43) Veröffentlichungstag der Anmeldung:
**11.07.79 Patentblatt 79/14**

(45) Bekanntmachung des Hinweises auf die Patenterteilung:
**17.09.80 Patentblatt 80/19**

(84) Benannte Vertragsstaaten:
**CH DE FR GB IT NL**

(56) Entgegenhaltungen:
**JOURNAL OF THE AMERICAN CHEMICAL SOCIETY, Vol. 74, 5.November '52, Seiten 5.527—9**
**PETE D. GARDNER et al. "A new approach to δ-Phenylvaleric acids"**

(73) Patentinhaber: **HAARMANN & REINER GMBH Postfach 138**
**D - 3450 Holzminden (DE)**

(72) Erfinder: **Oediger, Hermann, Dr.**
**Roggendorfstrasse 51**
**D - 5000 Köln 80 (DE)**
**Schulze, Andreas, Dr.**
**Jakob-Böhme-Strasse 19**
**D - 5000 Köln 80 (DE)**

(74) Vertreter: **Dill, Erwin, Dr.**
**c/o BAYER AG Zentralbereich Patente Marken und Lizenzen Bayerwerk**
**D - 5090 Leverkusen 1 (DE)**

**EP 0 002 734 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Verfahren zur Herstellung von Piperonylidencrotonsäureamiden

Die vorliegende Erfindung betrifft ein neues Verfahren zur Herstellung von Piperonylidencrotonsäureamiden.

Piperonylidencrotonsäureamide finden eine vielfältige technische Verwendung. So ist Piperonylidencrotonsäurepiperidid (Trivialname: Piperin) ein wichtiger Würzstoff. Er stellt das Schärfeprinzip des schwarzen Pfeffers dar (Chromatographia Band 8 (1975), Seiter 342—344). Als Würzstoffe eignen sich ferner zur Geschmacksabrundung von Pfefferzubereitungen Piperonylidencrotonsäurepyrrolidid auch Piperylin genannt (Chem. Ber. Ed. 103 (1970), Seiten 3752—3770) und Piperonylidencrotonsäureisobutylamid auch Piperlonguminin genannt (Tetrahedron Bd. 23 (1967), Seiten 1769—1781). Piperin findet ferner Verwendung als Zusatz zu germiziden Zubereitungen (US—PS 2 085 064). Piperonylidencrotonsäureamide eignen sich auch als Insektizide oder Insektizid-Synergisten (US—PS 2 487 179; Contrib. Boyce Thompson Inst. Bd. 13 (1945) Seiten 433—442; UdSSR-Pat. 222 056; DT—OS 2 413 756). Weiterhin eignet sich Piperin auch als Analeptikum bei Morphin- oder Barbituratvergiftungen (J. Res. Indian Med. Bd. 8 (1973), Seiten 1—9 und Bd. 9 (1974) Seiten 17—22).

Es sind deshalb bereits verschiedene Verfahren zur Herstellung von Piperonylidencrotonsäureamiden insbesondere von Piperin vorgeschlagen worden. So wird nach einem Herstellungsverfahren zunächst Piperonal in einem dreistufigen Verfahren in Piperonylidenacetaldehyd überführt, dieser mit Malonsäurehalbester zum Piperonylidencrotonsäureester kondensiert und dieser über weitere drei Stufen mittels Piperidin in das entsprechende Piperidid überführt (Chem. Ber. Bd. 108 (1975), Seiten 95—108).

Dieses Verfahren ist jedoch wegen seiner vielen Verfahrensschritte und der unbefriedigenden Ausbeuten — die Gesamtausbeute beträgt lediglich 41% — unwirtschaftlich und kommt deshalb für eine Darstellung von Piperin in technischem Naßstab nicht in Betracht.

Weiterhin hat man Piperin durch Kondensation von Piperonylidenacetaldehyd mit Piperidonocarbonylmethyl-triphenylphosphoniumjodid hergestellt (Pharm. Chem. J. Bd. 5 (1971), Seiten 462 bis 464). Diese Herstellung hat jedoch den Nachteil, daß die als Reaktionskomponenten einzusetzenden Verbindungen ihrerseits erst durch mehrstufige Verfahren hergestellt werden müssen und daß bei der Kondensation große Mengen Triphenylphosphinoxid entstehen, die sich nur umständlich vom Piperin abtrennen lassen. Auch dieses Verfahren ist daher für eine Piperin-Synthese in technischem Naßstab ungeeignet.

Es wurde nun gefunden, daß man bekannte und neue Piperonylidencrotonsäureamide der Formel

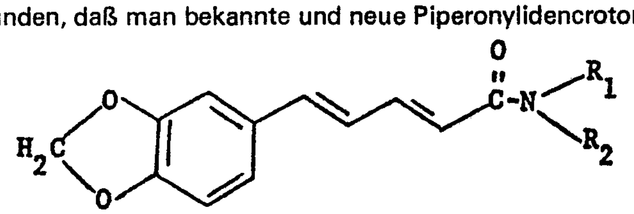
I

in der

$R_1$ und $R_2$ unabhängig voneinander für Wasserstoff, oder einen gegebenenfalls substituierten aliphatischen, araliphatischen oder aromatischen Kohlenwasserstoffrest stehen oder gemeinsam mit dem Stickstoffatom einen heterocyclischen Ring bilden, mit der Maßgabe, daß $R_1$ und $R_2$ nicht gleichzeitig Wasserstoff bedeuten,

auf einfache Weise in ausgezeichneter Ausbeute herstellen kann, wenn man Piperonal mit Crotonsäureamiden der Formel

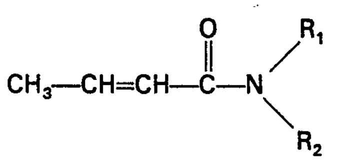
II

in der

$R_1$ und $R_2$ die vorstehend angegebene Bedeutung haben, in Gegenwart von Hydroxiden der Formel

$$A^+ \ OH^- \qquad III$$

in der

$A^+$ für eine quartäre Ammonium- oder Phosphoniumgruppe oder einen Alkalimetallkomplex mit neutralen organischen Komplexliganden steht,

und unter den Reaktionsbedingungen inerten, polaren aprotischen oder polaren, sterisch gehinderten protischen Lösungsmitteln umsetzt.

Das erfindungsgemäße Verfahren zeichnet sich gegenüber den aus dem Stand der Technik bekannten Verfahren durch eine wesentlich vereinfachte Arbeitsweise (wenige Reaktionsstufen, Verwendung technisch ohne besondere Vorsichtsmaßnahmen handhabbarer Kondensationsmittel) und durch erheblich verbesserte Ausbeuten aus. Nach dem erfindungsgemäßen Verfahren lassen sich Piperonylidencrotonsäureamide ohne Schwierigkeiten in technischem Maßstab herstellen. Die Verbindungen fallen in hoher Reinheit an.

Es war zwar bereits bekannt, Aldehyde mit 3-Methylbutencarbonsäureestern, 3-Methylbutencarbonsäureamiden oder Crotonsäureestern zu kondensieren (US—PS 2 951 853; Chem. Ber. Bd. 106 (1973), Seiten 2643—2647). Die Kondensationen wurden jedoch unter Verwendung mindestens äquimolarer Mengen starker Kondensationsmittel wie Alkalimetallen, Alkaliamiden, Alkalihydriden, oder metallorganischen Verbindungen wie Phenylnatrium oder Triphenylmethylkalium, bezogen auf die Menge Alkensäureester und völligem Ausschluß von Wasser durchgeführt.

Es war daher überraschend, daß die erfindungsgemäße Umsetzung bereits mit katalytischen Mengen als schwächer wirksam bekannter Kondensationsmittel, wie Ammonium- oder Phosphoniumhydroxiden oder Hydroxiden von Alkalikomplexen mit neutralen organischen Komplexliganden, auch ohne Ausschluß von Wasser in hohen Ausbeuten verläuft. Im Hinblick darauf, daß Piperonal in seiner Reaktionsfähigkeit mit 4-Methoxybenzaldehydvergleichbar ist, und da bekannt war, daß desaktivierte Aldehyde wie 4-Methoxybenzaldehyd mit Crotonsäureäthylester selbst in Gegenwart starker Kondensationsmittel wie Natriumamid nur in 12%iger Ausbeute zum gewünschten Kondensationsprodukt reagieren, (Chem. Ber. Bd. 106 (1973), Seiten 2643—2647) war zu erwarten, daß unter den erfindungsgemäßen Reaktionsbedingungen keinerlei Umsatz stattfinden würde.

Es war ferner bekannt, daß Piperonal mit Äthylidenmalonsäureester in Gegenwart von Kaliumhydroxid und Äthanol oder in Gegenwart eines großen Überschusses an Benzyltrimethylammoniumhydroxid und Methanol kondensiert (J. Am. Chem. Soc. Bd. 74 (1952), Seiten 5527—5529). Ersetzt man in dieser Reaktion jedoch den als sehr reaktionsfähig bekannten Äthylidenmalonsäureester durch die wesentlich reaktionsträgeren Crotonsäureamide, so fallen die Ausbeuten an Kondensationsprodukt derartig stark ab (auf etwa 10—15% der Theorie), so daß diese Reaktion für die Herstellung von Piperonylidencrotonsäureamiden uninteressant ist.

Bei den erfindungsgemäß zu verwendenden quartären Ammonium- und Phosphoniumhydroxiden handelt es sich um Verbindungen der Formel

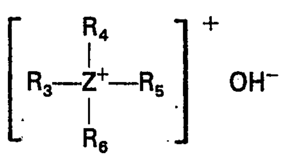

IV

in der

Z für Phosphor oder, vorzugsweise, für Stickstoff steht und

$R_3$, $R_4$, $R_5$, u. $R_6$ unabhängig voneinander gegebenenfalls substituiertes Alkyl, Cycloalkyl, Aralkyl oder Aryl bedeuten, zwei benachbarte Reste $R_3$, $R_4$, $R_5$, $R_6$ zusammen mit dem Zentralatom Z und gegebenenfalls weiteren Heteroatomen einen Heterocyclus bilden oder, für den Fall, daß Z Phosphor bedeutet, bis zu drei der Reste $R_3$, $R_4$, $R_5$, $R_6$ für eine Dialkylaminogruppe stehen.

Für $R_3$, $R_4$, $R_5$ und $R_6$ seien beispielsweise genannt:

Als Alkylreste vor allem $C_1$—$C_{18}$-Alkylreste, wie der Methyl-, Äthyl-, Propyl-, sec. Butyl-, Heptyl-, Hexyl-, i-Octyl-, Dodecyl- und Octadecyl-Rest; als Cycloalkylreste gegebenenfalls durch $C_1$—$C_4$-Alkylreste substituierte Cyclopentyl- und insbesondere Cyclohexylreste; als Aralkylreste gegebenenfalls durch $C_1$—$C_4$-Alkylreste oder Methoxygruppen oder Halogen substituierte Benzylreste; als Arylrest vor allem durch $C_1$—$C_4$-Alkyl- oder $C_1$—$C_2$-Alkoxygruppen oder Halogenatome substituierte Phenylreste.

Als Beispiele für Heterocyclen, die zwei benachbarte Reste $R_3$, $R_4$, $R_5$, $R_6$ zusammen mit dem Zentralatom Z und gegebenenfalls weiteren Heteroatomen wie Sauerstoff, Schwefel oder Stickstoff bilden können, seien vor allem 5- oder 6-gliedrige Heterocyclen, wie der Pyrrolidin-, Piperidin- oder Morpholinring genannt.

Als Dialkylaminogruppe sei vor allem die Dimethylaminogruppe genannt.

Als Vertreter der erfindungsgemäß zu verwendenden Ammonium- und Phosphoniumhydroxide seien beispielsweise genannt:

Octylbenzyldimethylammoniumhydroxid,
Octadecylbenzyldimethylammoniumhydroxid,
Trimethylphenylphosphoniumhydroxid,
Benzyltriäthylammoniumhydroxid,
Benzyltributylammoniumhydroxid,

Benzyltrimethylammoniumhydroxid,
Cetyltrimethylammoniumhydroxid,
Methyltributylammoniumhydroxid,
Tetradecyltrimethylammoniumhydroxid,
Phenyltrimethylammoniumhydroxid,
Tetrabutylammoniumhydroxid,
Tetraäthylammoniumhydroxid,
Tetramethylammoniumhydroxid,
Benzyldodecyldimethylammoniumhydroxid,
Dimethylphenylbenzylammoniumhydroxid,
Methyltrioctylammoniumhydroxid,
Tetramethylphosphoniumhydroxid,
Tetraäthylphosphoniumhydroxid,
Tetrabutylphosphoniumhydroxid,
Tripropylbutylphosphoniumhydroxid,
Benzyltrimethylphosphoniumhydroxid,
Tris-(dimethylamino)-methylphosphoniumhydroxid,
Tributylmethylphosphoniumhydroxid,
β-Hydroxyäthyltrimethylammoniumhydroxid,
β-Hydroxyäthyltriäthylammoniumhydroxid.

Die Verbindungen sind bekannt oder können nach bekannten Verfahren aus den entsprechenden Salzen hergestellt werden (s. z.B. Houben-Weyl, Methoden der Organischen Chemie, 4. Auflage, Band 12/1, (1963), Seite 106).

Bei den erfindungsgemäß zu verwendenden Alkalimetallkomplexen handelt es sich um Alkalimetallkomplexe der unter den Trivialbezeichnungen "Kronenäther", "Cryptanden" und "Podanden" bekannten neutralen organischen Komplexliganden. Die Verbindungen als solche sind bekannt oder können nach bekannten Methoden hergestellt werden (s. z.B. US—PS 3,562,295, 3,860,611 und 3,966,766).

Als "Kronenäther" werden allgemein mittel- bis vielgliedrige cyclische Polyäther bezeichnet, in denen Saurestoff-Donatoratome meist durch Äthanolbrücken verbunden sind und die ein oder mehrere ankondensierte Benzol- oder Cyclohexanringe enthalten können (s. J. Am. Chem. Soc. 89, 7017 (1969); Chem. Rev. 74, 351 (1974); Chem. Commun. 1976, 295).

Als "Cryptanden" werden allgemein dreidimensionale mittelbis vielgliedrige di-, tri- und tetracyclische Aminopolyäther bezeichnet (s. Endeavour 1971, 142; J. Am. Chem. Soc. 97, 6700 (1975)).

Bei den "Podanden" handelt es sich um mit Kronenäthern verwandte, jedoch nicht cyclische Neutral-Liganden (s. Tetrahedron Let. 1975, 2415).

Als Alkalimetallionen kommen für die Alkalimetallkomplexe Lithium- und insbesondere Kalium- und Natriumionen in Betracht.

Als Vertreter der erfindungsgemäß zu verwendenden Alkalimetallkomplexe mit Kronenäthern, Cryptanden und Podanden seien beispielsweise genannt:

Die Kaliumkomplexe mit
1,4,7,10,13,16-Hexaoxacyclooctadecan (18-Krone-6),
1,13-Bis-(8-chinolyl)-1,4,7,10,13-pentaoxatridecan,
4,7,13,16,21,24-Hexaoxa-1,10-diazabicyclo[8,8,8]-hexacosan,
5,6-Benzo-4,7,13,16,21,24-hexaoxa- 1,10-diazabicyclo[8,8,8]-hexacosan,
5,6,14,15-Dibenzo-4,7,13,16,21,24-hexaoxa- 1,10-diazabicyclo[8,8,8]-hexacosan,
5-Decyl-4,7,13,16,21,24.hexaoxa-1,10-diazabicyclo[8,8,8]-hexacosan,
1,4,7,14,23-Pentaoxa[7,2]-orthocyclo[2](2,6)-pyridinophan (Dibenzopyridino-18-krone-6),
1,4,7,14,17,20-Hexaoxa[7,7]orthocyclophan (Dibenzo-18-krone-6),
2,5,8,15,18,21-Hexaoxatricyclo[20,4,0,0$^{9,14}$]hexacosan (Dicyclohexyl-18-krone-6).

Die Natriumkomplexe mit
1,4,7,10,13-Pentaoxacyclopentadecan (15-Krone-5),
4,7,13,16,21-Pentaoxa-1,10-diazabicyclo[8,5,5]tricosan.

Die Lithiumkomplexe mit
1,4,7,10-Tetraoxacyclododecan (12-Krone-4),
4,7,13,18-Tetraoxa-1,10-diazabicyclo[8,5,5]-eicosan.

Die erfindungsgemäß zu verwendenden Hydroxide der Formel III können als solche, d.h. in isolierter Form eingesetzt werden. Sie können jedoch auch erst im Reaktionsgemisch erzeugt werden, z.B. durch Zugabe entsprechender Mengen Natrium- oder Kaliumhydroxid zu den Ammonium- oder Phosphoniumsalzen oder zu den neutralen organischen Komplexliganden.

Die Hydroxide der Formel III werden im allgemeinen in Mengen von 0,04—0,2 Mol, vorzugsweise 0,05—0,1 Mol je Mol Piperonal angewendet.

Als unter den Reaktionsbedingungen inerte polare aprotische Lösungsmittel seien beispielsweise genannt:

schwächer polare aprotische Lösungsmittel wie aromatische Kohlenwasserstoffe, z.B. Toluol oder Xylol, isbesondere aber die stärker polaren aprotischen Lösungsmittel wie aliphatische, araliphatische oder cyclische Äther, z.B. Dibutyläther, Äthylenglykoldimethyläther, Diäthylenglykoldimethyläther, Diäthylenglykoldiäthyläther, Anisol, Tetrahydrofuran, Dioxan; Säureamide, z.B. Dialkylformamide oder Dialkylacetamide wie Dimethylformamid, Dimethylacetamid oder N-Methyl-2-pyrrolidinon; Tetraalkylharnstoffe wie Tetramethylharnstoff; Sulfoxide, z.B. Dialkylsulfoxide wie Dimethylsulfoxid und Tetrahydrothiophen-1-oxid; Sulfone wie Tetrahydrothiophen-1,1-dioxid; Hexaalkylphosphorsäurediamide wie Hexamethylphosphorsäuretriamid oder 1-Methyl-1-oxophospholin; tertiäre Amine wie Tributylamin, Dimethylanilin oder Dimethylbenzylamin.

Als polare, sterisch gehinderte protische Lösungsmittel seien vor allem Tertiär-butanol und 1,1-Dimethylhexanol genannt.

Die Mengen, in denen die vorstehend genannten organischen Lösungsmittel eingesetzt werden, können in weiten Grenzen schwanken; im allgemeinen hat es sich bewährt, je Mol Piperonal 50—500, vorzugsweise 100—350 ml Lösungsmittel einzusetzen.

Eine Erhöhung der Lösungsmittelmenge über die angegebene Höchstmenge von 500 ml hinaus ist möglich, bringt im allgemeinen aber keinen Vorteil.

Die erfindungsgemäße Kondensation wird im allgemeinen bei Temperaturen zwischen 20°C und 100°C, vorzugsweise zwischen 50°C und 80°C vorgenommen.

Die Reaktion kann bei Normaldruck oder erhöhtem Druck druchgeführt werden. Im allgemeinen arbeitet man bei Normaldruck. Vorzugswiese arbeitet man in einer Intergasatmosphäre, beispielsweise unter Stickstoff oder Argon.

In dem erfindungsgemäßen Verfahren setzt man im allgemeinen die beiden Reaktionspartner in etwa äquimolaren Mengen ein; es hat sich bewährt, je Mol Piperonal 1 bis 1,2 Mol Crotonsäureamid, vorzugsweise 1 bis 1,1 Mol Crotonsäureamid zu verwenden.

Die erfindungsgemäß hergestellten Piperonylidencrotonsäureamide werden aus dem Reaktionsgemisch durch Abdampfen des Lösungsmittels isoliert und beispielsweise durch Waschen mit Wasser von löslichen Verunreinigungen befreit. Die Verbindungen können durch Umkristallisieren gereinigt werden.

Der Verlauf des erfindungsgemäßen Verfahrens sei an folgendem Reaktionsschema erläutert:

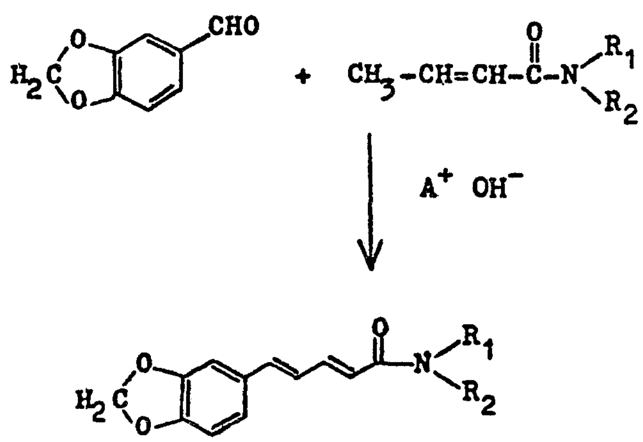


Für $R_1$ und $R_2$ seien beispielsweise genannt:

Als gegebenenfalls substituierte aliphatische Kohlenwasserstoffreste vor allem $C_1$—$C_6$-Alkyl-, $C_1$—$C_6$-Alkenyl- und 5- und 6-gliedrige Cycloalkylreste. Beispielsweise der Methyl-, Äthyl-, n-Propyl-, i-Propyl-, n-Butyl-, sec.-Butyl-, i-Pentyl-, n-Hexyl-, i-Hexyl-, Allyl-, Cyclopentyl- und Cyclohexylrest, ferner durch $C_1$—$C_4$-Alkylgruppen substituierte Cyclopentyl und Cyclohexylreste wie der 4-Methyl- und 2,4-Dimethyl-cyclohexylrest. Als Substituenten für die Alkylreste kommen vor allem Halogenatome wie das Chloratom und die Hydroxy-Gruppe in Betracht. Beispiele für substituierte Alkylreste: der 2-Chloräthyl- und 2-Hydroxyäthyl-Rest.

Als gegebenenfalls substituierte araliphatische und aromatische Kohlenwasserstoffreste, vor allem der Benzyl- und Phenylrest und durch Halogenatome, z.B. Chlor- oder Bromatome, durch $C_1$—$C_4$-Alkyl- und $C_1$—$C_4$-Alkoxygruppen substituierte Benzyl- und Phenylreste wie der 4-Methyl- und 3-Chlorbenzyl-Rest und der 3-Chlor-, 2,4-Dichlor-, 2-Brom-4-methyl-, 4-Äthyl- und der 4-Methoxyphenyl-Rest.

Als Heterocyclen, die $R_1$ und $R_2$ zusammen mit dem Amidstickstoff bilden können vor allem 5- bis 7-gliedrige, gegebenenfalls weitere Heteroatome wie Sauerstoff, Schwefel oder Stickstoff enthaltende Heterocyclen wie der Piperidin-, Pyrrolidin-, Morpholin- und Hexamethylenimin-Ring. Diese

Heterocyclen können auch substituiert sein. z.B. durch $C_1$—$C_4$-Alkylgruppen: z.B. 2-, 3- und 4-Methylpiperidin, 2,3-, 2,4- und 2,6-Dimethylpiperidin, 2-Äthyl-piperidin, 2,4,6-Trimethylpiperidin.

Die erfindungsgemäß als Ausgangsverbindungen zu verwendenden Crotonsäureamide sind bekannt oder können nach an sich bekannten Verfahren hergestellt werden, beispielsweise aus Crotonsäurechlorid und den entsprechende Aminen (Helv. Chim. Acta Bd. 38 (1955), Seiten 1085—1095) oder aus den entsprechenden Crotonsäureammoniumsalzen entweder durch Abspaltung von Wasser bei erhöhter Temperatur, gegebenenfalls in Gegenwart saurer Katalysatoren oder durch Umsetzung mit anorganischen Säurehalogeniden, z.B. Thionylchlorid (Houben-Weyl, Methoden der organischen Chemie, Bd. XI/2 Seiten 3—9, Thieme Verlag Stuttgart 1958).

Als Vertreter der erfindungsgemäß umzusetzenden Crotonsäureamide seien beispielsweise genannt:

Crotonsäurepiperidid,
Crotonsäurepyrrolidid,
Crotonsäuremorpholid,
Crotonsäurehexamethylenimid,
Crotonsäure-(2-methylpiperidid),
Crotonsäure-(3-methylpiperidid),
Crotonsäure-(4-methylpiperidid),
Crotonsäure-(2-pentylpiperidid),;
Crotonsäure-(4-entylpiperidid),
Crotonsäure-(2,4,6-trimethylpiperidid),
Crotonsäure-(2,6-dimethylpiperidid),
Crotonsäure-(2,4-dimethylpiperidid),
Crotonsäure-(2-äthylpiperidid),
Crotonsäure-(2,3-dimethylpiperidid),
Crotonsäuremethylamid,
Crotonsäureäthylamid,
Crotonsäurepropylamid,
Crotonsäureallylamid,
Crotonsäurebutylamid,
Crotonsäureisobutylamid,
Crotonsäure-isopentylamid,
Crotonsäure-cyclohexylamid,
Crotonsäure-(3-äthyl-heptylamid),
Crotonsäurebenzylamid,
Crotonsäure-(3,4-methylendioxy-anilid),
Crotonsäure-anilid,
Crotonsäure-(2-brom-4-methyl-anilid),
Crotonsäure-dimethylamid,
Crotonsäure-diäthylamid,
Crotonsäure-dipropylamid,
Crotonsäure-diisopropylamid,
Crotonsäure-diallylamid,
Crotonsäure-dibutylamid,
Crotonsäure-diisobutylamid,
Crotonsäure-dicyclohexylamid,
Crotonsäure-(di-β-chloräthyl-amid),
Crotonsäure-(di-β-hydroxyäthyl-amid).

Als Vertreter der nach dem erfindungsgemäßen Verfahren herstellbaren Piperonylidencrotonsäureamide seien beispielsweise genannt:

Piperonylidencrotonsäurepiperidid,
Piperonylidencrotonsäurepyrrolidid,
Piperonylidencrotonsäuremorpholid,
Piperonylidencrotonsäurehexamethylenimid,
Piperonylidencrotonsäure-(2-methylpiperidid),
Piperonylidencrotonsäure-(3-methylpiperidid),
Piperonylidencrotonsäure-(4-methylpiperidid),
Piperonylidencrotonsäure-(2-pentylpiperidid),
Piperonylidencrotonsäure-(4-pentylpiperidid),
Piperonylidencrotonsäure-(2,4,6-trimethylpiperidid),
Piperonylidencrotonsäure-(2,6-dimethylpiperidid),
Piperonylidencrotonsäure-(2,4-dimethylpiperidid),

Piperonylidencrotonsäure-(2-äthylpiperidid),
Piperonylidencrotonsäure-(2,3-dimethylpiperidid),
Piperonylidencrotonsäuremethylamid,
Piperonylidencrotonsäureäthylamid,
Piperonylidencrotonsäurepropylamid,
Piperonylidencrotonsäureallylamid,
Piperonylidencrotonsäureisobutylamid,
Piperonylidencrotonsäure-isopentylamid,
Piperonylidencrotonsäure-cyclohexylamid,
Piperonylidencrotonsäure-(3-äthyl-heptylamid),
Piperonylidencrotonsäurebenzylamid,
Piperonylidencrotonsäure-(3,4-methylendioxy-anilid),
Piperonylidencrotonsäure-anilid,
Piperonylidencrotonsäure-(2-brom-4-methyl-anilid),
Piperonylidencrotonsäure-dimethylamid,
Piperonylidencrotonsäure-diäthylamid,
Piperonylidencrotonsäure-di-propylamid,
Piperonylidencrotonsäure-diisopropylamid,
Piperonylidencrotonsäure-diallylamid,
Piperonylidencrotonsäure-dibutylamid,
Piperonylidencrotonsäure-diisobutylamid,
Piperonylidencrotonsäure-dicyclohexylamid,
Piperonylidencrotonsäure-(di-$\beta$-chloräthyl-amid),
Piperonylidencrotonsäure-(di-$\beta$-hydroxyäthyl-amid).

Bei den in den folgenden Beispielen angegebenen Teilen handelt es sich um Gewichtsteile, sofern nichts anderes angegeben wurde.

Beispiel 1

Die Lösung von 150 Teilen (1 Mol) Piperonal und 170 Teilen (1,1 Mol) Crotonsäurepiperidid in 100 Volumenteilen Dimethylsulfoxid wird bei 25°C unter Stickstoff mit 23 Teilen Triäthylbenzylammoniumchlorid und 10 Teilen 50%-iger wässriger Kalilauge versetzt. Die Reaktonsmischung wird 15 Minuten bei 25°C, anschließend 2 Stunden bei 60 bis 65°C gerührt und danach im Vakuum von Lösungsmittel befreit. Der Rückstand wird mit 400 Volumenteilen Wasser versetzt. Das ausgefallene Reaktionsprodukt wird abgesaugt, mit Wasser gewaschen und getrocknet.

Ausbeute: 276 Teile Roh-Piperonylidencrotonsäurepiperidid.

Nach dem Umkristallisieren aus Äthylacetat beträgt die Ausbeute an reinem Piperonylidencrotonsäurepiperidid 251 Teile (= 88% der Theorie). Schmelzpunkt 129—130°C.

Beispiele 2—8

1 Mol Piperonal wird mit 1,1 Mol eines in der nachstehenden Tabelle 1 angegebenen Crotonsäureamides in Gegenwart von 0,09 Mol Triäthylbenzylammoniumhydroxid unter den in Beispiel 1 angegebenen Bedingungen umgesetzt. Es werden die in Tabelle 1 aufgeführten Piperonylidencrotonsäureamid in den ebenfalls in der Tabelle angegebenen Ausbeuten erhalten.

TABELLE 1

Formeln der eingesetzten Crotonsäureamide (a)
und der aus ihnen erhaltenen Piperonylidencrotonsäureamide (b)

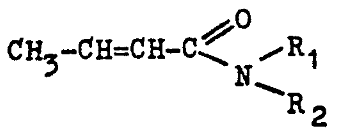

(a)

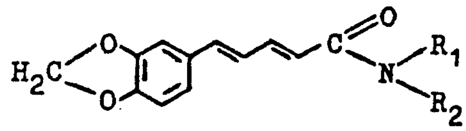

(b)

| Beispiel Nr. | 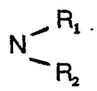 | Ausbeute in % der Theorie | Fp in °C |
|---|---|---|---|
| 3 | 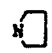 | 83 | 142 – 143 |
| 4 | 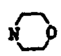 | 81 | 167 – 168 |
| 5 | $HN-CH_2-CH(CH_3)_2$ | 78 | 165 – 166 |
| 6 | $N(C_2H_5)_2$ | 84 | 94 – 95 |
| 7 | $N(CH_2-CH=CH_2)_2$ | 86 | 75 – 76 |
| 8 | 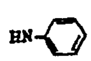 | 80 | 197 – 198 |

Beispiele 9—15

1 Mol Piperonal wird mit 1,1 Mol Crotonsäurepiperidid in Gegenwart von 0,09 Mol eines inder nachstehenden Tabelle 2 angegebenen Hydroxids unter den in Beispiel 1 beschriebenen Reaktionsbedingungen zu Piperonylidencrotonsäurepiperidid umgesetzt. Es werden die ebenfalls in Tabelle 2 angegebenen Ausbeuten erhalten.

TABELLE 2

Formel der eingesetzten Hydroxide

$A^{\oplus} OH^{\ominus}$

| Beispiel Nr. | $A^{\oplus}$ | Ausbeute in % der Theorie |
|---|---|---|
| 9 | $(CH_3)_3N-$ 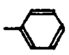 | 82 |
| 10 | $(CH_3)_2N-(CH_2)_{11}-CH_3$ <br> $CH_2-$ 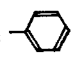 | 74 |
| 11 | $(CH_3)_3N-CH_2-$ 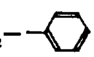 | 76 |
| 12 | $(C_2H_5)_4N$ | 80 |
| 13 | $(C_4H_9)_4N$ | 78 |
| 14 | $(C_8H_{17})_3N-CH_3$ | 73 |
| 15 | 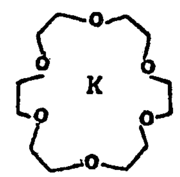 | 86 |

Beispiele 16—24

1 Mol Piperonal wird mit 1,1 Mol Crotonsäurepiperidid in Gegenwart von 0,09 Mol Triäthylbenzylammoniumhydroxid in 100 Volumenteilen eines der in der nachfolgenden Tabelle 3 angegebenen Lösungsmittel unter den in Beispiel 1 beschriebenen Bedingungen zu Piperonylidencrotonsäurepiperidid umgesetzt. Es werden die ebenfalls in Tabelle 3 angegebenen Ausbeuten erzielt.

TABELLE 3

| Beispiel Nr. | Lösungsmittel | Ausbeute in % der Theorie |
|---|---|---|
| 16 | Dimethoxyäthan | 75 |
| 17 | Diäthylenglykoldimethyläther | 81 |
| 18 | tert.-Butanol | 69 |
| 19 | Tetrahydrothiophen-1,1-dioxid | 71 |
| 20 | N-Methylpyrrolidinon | 72 |
| 21 | Tetrahydrofuran | 68 |
| 22 | Dimethylbenzylamin | 76 |
| 23 | Dimethylacetamid | 73 |
| 24 | Anisol | 80 |

Beispiel 25

Die Lösung von 15 Teilen (0,1 Mol) Piperonal und 17 Teilen (0,11 Mol) Crotonsäurepiperidid in 10

Volumenteilen Toluol wird bei 25°C unter Stickstoff zunächst mit 2,6 Teilen 18-krone-6 und dann mit einem Teil 50%-iger wässringer Kalilauge versetzt. Die Reaktionsmischung wird 15 Minuten bei 25°C, dann 2 Stunden bei 60 bis 65°C gerührt und anschließend im Vakuum vom Lösungsmittel befreit. Der Rückstand wird mit 40 Volumenteilen Wasser versetzt. Das ausgefallene Reaktionsprodukt wird abgesaugt, mit Wasser gewaschen und getrocknet.

Ausbeute: 24 Teile Roh-Piperonylidencrotonsäurepiperidid.

Nach dem Umkristallisieren aus Äthylacetat beträgt die Ausbeute an reinem Piperonylidencrotonsäurepiperidid 19,2 Teile (= 56% der Theorie). Schmelzpunkt 129—130°C.

Beispiel 26

Die Lösung von 15 Teilen (0,1 Mol) Piperonal und 17 Teilen (0,11 Mol) Crotonsäurepiperidid in 10 Volumenteilen Dimethylsulfoxid wird bei 25°C unter Stickstoff zunächst mit 2,3 Teilen Triäthylbenzylammoniumchlorid und anschließend mit einem Gewichtsteil 40%-iger wässriger Natronlauge versetzt. Die Reaktionsmischung wird 15 Minuten bei 25°C, dann 2 Stunden bei 60 bis 65°C gerührt und anschließend im Vakuum vom Lösungsmittel befreit. Der Rückstand wird mit 40 Volumenteilen Wasser verrührt. Das ausgefallene Reaktionsprodukt wird abgesaugt, mit Wasser gewaschen und getrocknet.

Ausbeute an Roh-Piperonylidencrotonsäurepiperidid: 27 Teile.

Nach dem Umkristallisieren aus Äthylacetate beträgt die Ausbeute an reinem Piperonylidencrotonsäurepiperidid 23 Teile (= 80% der Theorie). Schmelzpunkt: 129—130°C.

Beispiel 27

Die Lösung von 15 Teilen (0,1 Mol) Piperonal und 17 Teilen (0,11 Mol) Crotonsäurepiperidid in 10 Volumenteilen Anisol wird bei 25°C unter Stickstoff mit 4 Teilen einer 40%-igen wässrigen Lösung von Benzyltrimethylammoniumhydroxid versetzt. Die Reaktionsmischung wird 2 Stunden bei 60 bis 65°C gerührt und anschließend im Vakuum vom Lösungsmittel befreit. Der Rückstand wird mit 40 Volumenteilen Wasser verrührt. Das angefallene Reaktionsprodukt wird abgesaugt, mit Wasser gewaschen und getrocknet.

Ausbeute an Roh-Piperonylidencrotonsäurepiperidid: 27 Teile.

Nach dem Umkristallisieren aus Äthylacetate beträgt die Ausbeute an reinem Piperonylidencrotonsäurepiperidid 22,5 Teile (= 79%). Schmelzpunkt: 129—130°C.

Beispiel 28

Die Lösung von 150 Teilen (1 Mol) Piperonal, 170 Teilen (1,1 Mol) Crotonsäurepiperidid und 25 g Benzyldimethylphenylammoniumchlorid in 100 Volumenteilen Anisol wird bei 25°C unter Stickstoff mit 10 Gewichtsteilen einer 50%-igen Kalilauge versetzt. Die Reaktionsmischung wird 15 Minuten bei 25°C, dann 2 Stunden bei 60—65°C gerührt und anschließend im Vakuum vom Lösungsmittel befreit.

Der Rückstand wird mit 400 Volumenteilen Wasser verrührt. Das angefallene Reaktionsprodukt wird abgesaugt, mit Wasser gewaschen und getrocknet.

Ausbeute an Roh-Piperonylidencrotonsäurepiperidid: 279 Teile.

Nach dem Umkristallisieren aus Ethylacetat beträgt die Ausbeute an reinem Piperonylidencrotonsäurepiperidid 251 Teile (= 88% d. Th). Schmelzpunkt: 129—130°C.

Beispiel 29

Man verfährt wie in Beispiel 28, ersetzt jedoch das dort eingesetzte Benzyldimethylphenylammoniumchlorid durch 34 Gewichtsteile Benzyldodecyldimethylammoniumchlorid.

Ausbeute an reinem Piperonylidencrotonsäurepiperidid: 245 Teile (= 85% d. Th.). Schmelzpunkt: 129—130°C.

**Patentansprüche**

1. Verfahren zur Herstellung von Piperonylidencrotonsäureamiden der allgemeinen Formel I

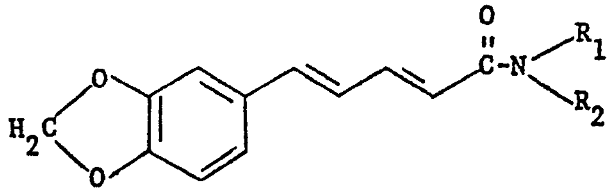

I

in der

$R_1$ und $R_2$ unabhängig voneinander für Wasserstoff, oder einen gegebenenfalls substituierten aliphatischen, araliphatischen oder aromatischen Kohlenwasserstoffrest stehen oder gemeinsam mit dem Stickstoffatom einen heterocyclischen Ring bilden, mit der Maßgabe, daß $R_1$ und $R_2$ nicht gleichzeitig Wasserstoff bedeuten, dadurch gekennzeichnet, daß man Piperonal mit Crotonsäureamiden der allgemeinen Formel

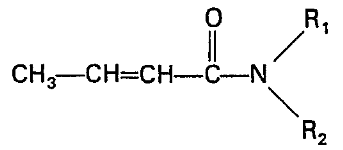

II

in der

$R_1$ und $R_2$ vorstehend angegebene Bedeutung haben, in Gegenwart von Hydroxiden der allgemeinen Formel III

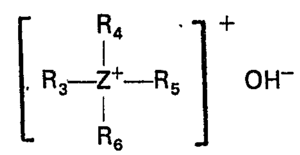

III

in der

Z für Phosphor oder, vorzugsweise, für Stickstoff steht,

$R_3$, $R_4$, $R_5$ und $R_6$ unabhängig voneinander gegebenenfalls substituiertes Alkyl, Cycloalkyl, Aralkyl oder Aryl bedeuten, zwei benachbarte Reste $R_3$, $R_4$, $R_5$, $R_6$ zusammen mit dem Zentralatom Z und gegebenenfalls weiteren Heteroatomen einen Heterocyclus bilden oder, für den Fall, daß Z Phosphor bedeutet, bis zu drei der Reste $R_3$, $R_4$, $R_5$, $R_6$ für eine Dialkylaminogruppe stehen, oder in Gegenwart von Hydroxiden der allgemeinen Formel IV

$$A^+ \, OH^- \qquad \text{IV}$$

in der

$A^+$ für einen Alkalimetallkomplex von Kronenäthern, Podanden oder Kryptanden steht, und unter den Reaktionsbedingungen inerten polaren aprotischen oder polaren, sterisch gehinderten protischen organischen Lösungsmitteln umsetzt.

2. Verfahren nach Anspruch 1, dadurch gekennzeichnet, daß man als inerte polare aprotische Lösungsmittel, stärker polare aprotische organische Lösungsmittel verwendet.

3. Verfahren nach Anspruch 1 und 2, dadurch gekennzeichnet, daß man die Hydroxide in Mengen von 0,04—0,2 Mol je Mol Piperonal einsetzt.

**Revendications**

1. Procédé de préparation d'amides pipéronylidènecrotoniques répondant à la formule générale:

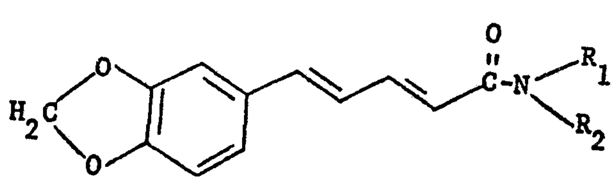

(I)

dans laquelle $R_1$ et $R_2$ représentent, indépendamment l'un de l'autre, l'hydrogène ou un reste hydrocarboné aliphatique, araliphatique ou aromatique éventuellement substitué ou bien forment ensemble et avec l'atome d'azote un hétérocycle, étant spécifié que $R_1$ et $R_2$ ne peuvent représenter simultanément l'hydrogène, ce procédé se caractérisant en ce que l'on fait réagir le pipéronal avec des amides crotoniques répondant à la formule générale:

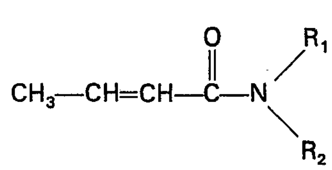

(II)

dans laquelle $R_1$ et $R_2$ ont la signification indiquée ci-dessus, en présence d'hydroxydes répondant à la formule générale:

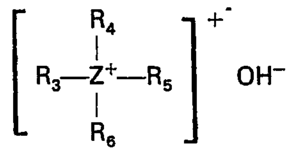

(III)

dans laquelle Z représente le phosphore ou, de préférence, l'azote, $R_3$, $R_4$, $R_5$ et $R_6$ représentent, indépendamment les uns des autres, des groupes alkyle, cycloalkyle, aralkyle ou aryle éventuellement substitués, deux restes $R_3$, $R_4$, $R_5$, $R_6$ voisins forment ensemble et avec l'atome central Z et, le cas échéant, d'autres hétéroatomes, un hétérocycle ou bien, dans le cas où Z représente le phosphore, jusqu'à 3 des restes $R_3$, $R_4$, $R_5$, $R_6$ représentent un groupe dialkylamino, ou en présence d'hydroxydes répondant à la formule générale:

$$A^{\oplus}OH^{\ominus} \qquad (IV)$$

dans laquelle $A^{\oplus}$ représente un complexe de métal alcalin d'éther en couronne, de podand ou de cryptand, et de solvants organiques polaires aprotoniques ou polaires protoniques faisant l'objet d'un empêchement stérique, inertes dans les conditions de la réaction.

2. Procédé selon la revendication 1, caractérisé en ce que l'on utilise en tant que solvants aprotoniques polaires inertes des solvants organiques aprotoniques fortement polaires.

3. Procédé selon la revendication 1 ou 2, caractérisé en ce que l'on utilise les hydroxydes en quantités de 0,04 à 0,2 mole par mole de pipéronal.

**Claims**

1. Process for the preparation of piperonylidenecrotonic acid amides of the formula I

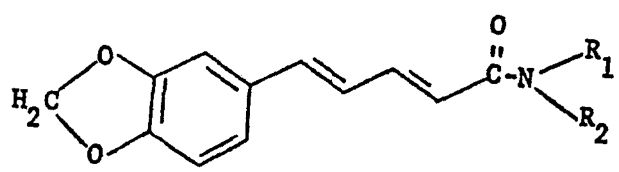

I

in which

$R_1$ and $R_2$ independently of one another represent hydrogen or an optionally substituted aliphatic, araliphatic or aromatic hydrocarbon radical, or together with the nitrogen atom, form a heterocyclic ring, with the proviso that $R_1$ and $R_2$ do not simultaneously denote hydrogen, characterised in that piperonal is reacted with crotonic acid amides of the formula

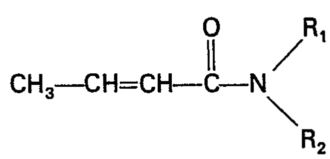

II

in which

$R_1$ and $R_2$ have the meaning indicated above, in the presence of hydroxides of the general formula III

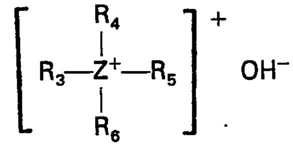

III

in which

Z represents phosphorus or, preferably, nitrogen,

$R_3$, $R_4$, $R_5$ and $R_6$ independently of one another denote optionally substituted alkyl, cycloalkyl, aralkyl or aryl, two adjacent radicals $R_3$, $R_4$, $R_5$ and $R_6$, together with the central atom Z and optionally

further hetero-atoms, form a heterocyclic ring or, in the case where Z denotes phosphorus, up to three of the radicals $R_3$, $R_4$, $R_5$ and $R_6$ represents a dialkylamino group, or in the presence of hydroxides of the general formula IV

$$A^+ \ OH^- \qquad IV$$

in which

$A^+$ represents an alkali metal complex of crown ethers, podands or cryptands, and polar aprotic or polar, sterically hindered protic organic solvents which are inert under the reaction conditions.

2. Process according to Claim 1, characterised in that relatively strongly polar aprotic organic solvents are used as the inert polar aprotic solvents.

3. Process according to Claim 1 and 2, characterised in that the hydroxides are employed in amounts of 0.04—0.2 mol per mol of piperonal.